# EUROPEAN PATENT SPECIFICATION

(11) **EP 2 051 772 B1**
(45) Date of publication and mention of the grant of the patent: **27.11.2013**
(21) Application number: 06769610.4
(22) Date of filing: 10.08.2006
(51) Int. Cl.: A61N 1/05, C23C 22/06, C23C 22/24, C23C 22/50

(54) **Implantable cardiac pacemaker lead**
Implantierbare Herzschrittmacherableitung
Conducteur implantable pour stimulateur cardiaque

(43) Date of publication of application: 29.04.2009
(73) Proprietor: St. Jude Medical AB, 175 84 Järfälla (SE)
(72) Inventor: ÖRNBERG, Andreas, 175 79 Järfälla (SE)
(74) Representative: Sjölander, Henrik
(86) International application number: PCT/SE2006/000943
(87) International publication number: WO 2008/018820

(56) References cited:
- US-A- 3 895 969
- US-A1- 2004 064 175
- US-A1- 2004 064 175
- US-A1- 2005 084 672
- US-A1- 2005 234 545
- US-B1- 6 558 732

## Description

### Field of the Invention

The invention relates generally to the field of passivation of metal surfaces. More specifically, the invention relates to passivation of metal surfaces on conductors used in cardiac leads which shelter polymeric electrical insulation adjacent thereto from metal and metal ions in the lead.

### Background of the Invention

Medical devices and components thereof often comprise significant amounts of metal or metal alloys. While metals are typically selected based on their biocompatibility, it is often the case that structural demands on the device require that materials which are not entirely biocompatible or which are not entirely compatible with other components of the device, particularly non-metal components, are employed. One example of this is in the field of cardiac pacemakers, where electrically conductive leads extend from the pacemaker to the heart of the recipient and comprise metal conductors with electrical insulation along their length.

In cardiac pacemaker lead bodies, the metals used are chosen based on a number of factors. Among these is fatigue-resistance; cobalt is known to improve the fatigue-resistance to an acceptable degree. The insulating materials are also carefully chosen and flexible polymer compositions, for example, polyether-based polyurethane are commonly used. One problem which can arise from this combination, though, is that the polymer insulation rapidly degrades if it is in direct contact with metal, particularly cobalt, and/or reactive species produced in the lead body. This catalytic degradation of the polymer insulation can lead to device failure or injury to the patient, both of which should be avoided whenever possible.

The degradation phenomenon is referred to as metal ion-induced oxidation or MIO. Some solutions to the problem of improving compatibility between metal-containing devices and insulating layers, i.e., ways to prevent or reduce MIO, have been disclosed. For example, a barrier formed of a material such as polytetrafluoroethylene (PTFE, a.k.a. TEFLON) or ethylene tetrafluoroethylene (ETFE) can be placed over the conductor or along the inside of the insulating layer to shield the insulation from the metal or metal alloys of the device. This process does have drawbacks; the addition of a third component in the device increases its size and makes the manufacturing process more complex and costly. Furthermore, creating bonds and joints in the resultant three-layer component is more complex.

With particular regard to cardiac pacemaker leads, researchers face intense pressure to maintain or even reduce the small diameter of present cardiac leads, meaning solutions that increase size are avoided whenever possible.

One way to reduce MIO without making drastic increases in final product dimensions is to provide a layer of conductive metal on the conductor, which metal is tolerated by the insulator. An example is platinum. This increases the overall dimensions by a lesser degree and typically does not present new issues during joining and bonding. However, the additional complex process steps to provide the layer and the materials themselves tend to be prohibitively expensive for many applications.

To meet the strict size demands and minimize manufacturing costs, other solutions have been proposed. For example, a voltage stabilizing additive (VSA) can be added to a polymeric insulating layer as taught in US 6,879,861. However, such polymers can be difficult and expensive to produce and the VSAs incorporated therein can leak into the patient, causing injury.

Alternatively, two layers of polymers with different characteristics can be used, the layer closest the metal being selected from those which are particularly resistant to MIO, while the outer layer is chosen based on qualities such as insulating ability and glidability. US 5,375,609, among others, provides examples of this configuration. While this might provide improved MIO-resistance in the outer insulating layer, the product cannot be optimized as the first layer must be considered, which may make the overall device less flexible, thicker, etc.

To help addressing the issues of flexibility, the inner layer of insulation can be a silicone layer, see US 5,628,774. However, this results in a device which still has undesirably large dimensions.

Thus, despite the advances described above, there remains a need in the art to improve known techniques and optimally provide improved devices which reduce the stress on the insulators without compromising device dimensions or other beneficial properties such as flexibility.

US 2005/0084672 discloses cobalt-chromium-molybdenum alloy wires for an implantable electrical lead. The wire is coated with a metal, ceramic or carbon to a thickness of about 100 nm or less to provide a non-reactive interface to polyurethane sheathing materials.

### Summary of the Invention

It is therefore an object of the present invention to provide improved implantable devices which comprise an electrically conductive metal or metal alloy having an outer surface.

This object is achieved with an implantable cardiac pacemaker lead as claimed in claim 1, comprising an electrically conductive metal or metal alloy having an outer surface; a passivated area on at least a portion of the metal or metal alloy outer surface; and an insulating layer having an inner surface configured to fit over at least part of said passivated area.

The electrically conductive metal or metal alloy comprises cobalt, and the insulating layer can be polymer-based, such as polyurethane. The improvement resides in providing the metal or metal alloy with a passivated surface, such that metal ion-induced oxidation (MIO) will be reduced.

The electrically conductive metal or metal alloy is the conductor in an elongated lead.

The metal conductor surface is depleted of Co atoms, and preferably it is also enriched in Cr atoms.

The passivated metal surface can be provided by chemical treatment, such as acid treatment.

The insulation layer can be provided around the entire passivated surface of the conductor.

A method of preparing an implantable medical device comprising an insulated conductor is provided, the method comprising providing an elongated metal or metal alloy conductor having an outer surface, treating the conductor with a suitable acid, such as HN03, and providing an insulation layer around at least a portion of the metal alloy conductor.

A method of protecting electrical insulation, suitably polymeric insulation, from metal ion-induced oxidation is provided, which comprises providing an electrically conductive metal or metal alloy having an outer surface, passivation treatment of at least a portion of the conductive metal or metal alloy outer surface, and positioning the polymeric electrical insulation on the passivated surface.

A kit for preparing an implantable medical device is provided, which comprises an electrically conductive metal or metal alloy, a means to provide the metal or metal alloy with a passivated area in the form of a layer or a film, and an insulating layer configured to fit on the electrically conductive metal or metal alloy.

### Detailed Description

For the purposes of this invention the term "passivation" shall be taken to mean a chemical treatment rendering the surface less prone to cause or contribute to metal ion-induced oxidation (MIO). Consequently a "passivated surface" or "passivated metal" shall be taken to mean a surface or metal that has been treated to exhibit a reduced degree of MIO in any application, preferably medical applications.

As noted above, the present invention provides a new configuration for implantable medical devices which allows for a slim profile while protecting insulating layers from MIO. This is achieved by providing a passivated surface on the medical device, suitably as a passivated layer or film, on at least part of the metal or metal alloy-containing device. This passivated surface layer or film shields the overlying insulating layer from MIO while having a negligible effect on the dimensions and mechanical properties of the device. The thickness of this film or layer is 1 nm - 20 nm, preferably 2 nm - 5 nm. In particular the passivated layer is depleted of Co and suitably enriched in Cr.

Acid treatments of metal surfaces for passivation purposes are known in the art. For exam- pie treatment of stainless steel by subjecting it to HNO₃ is a commonly used procedure to enhance corrosion resistance. Other known chemical treatments are e.g.
a) submersion in a chromic acid bath for 30 minutes at 46 °C,
b) submersion in a chromic acid bath for 60 minutes at 56 °C,
c) submersion in a tricresyl phosphate (TCP) bath for 2 days at 107 °C,
d) exposing the steel to citric acid solution, typically 4-10% by weight.

In a presently preferred embodiment HNO₃ is used as the acid for the chemical treatment. Suitably an aqueous solution of HNO₃ is used, the concentration of which is 5 - 30 % by weight. More preferably the concentration is 8-20% by weight, most preferred 10-15 % by weight.

The resultant passivated metal conductor has a layer of a modified alloy which is extremely thin, in the area of 1 nm to 20 nm. This allows the present invention to provide a device which does not measurably exceed current product dimensions. Furthermore, the passivated metal conductor maintains the advantageous properties of the underlying material, whether those are strength, flexibility, or other.

The devices of the present invention are particularly effective at shielding polyether-based polyurethane insulations from cobalt present in conductors in cardiac pacemaker leads. This is both because the passivated layer effectively depletes the surface of Co, but also because the passivated layer does not have a significant effect on product dimensions and mechanical properties, two factors that are exceedingly important with cardiac pacemaker leads.

### EXAMPLES

### Example 1 (passivation using HNO₃)

A passivation treatment of pacemaker lead coils was performed using 10,5% HNO₃ (aq) in order to improve the corrosion resistance as described below.

A cardiac pacemaker lead conductor was formed according to known methods from the fatigue-resistant electrical conducting material 35N LT (a non-magnetic, nickel-cobalt-chromium-molybdenum alloy available from FWM (Fort Wayne Metals, Indiana, USA); composition: approx 35% Co, 35% Ni, 20% Cr and 10% Mo by weight).

Sample lead coils (both inner and outer coils) were immersed in a 10,5 % (by weight) HNO₃ (aq) bath at a temperature of 35°C for a time of 150 minutes. Stirring could be beneficial.

However, the treatment can be performed at different temperatures. The temperature could be as low as 0°C but suitably not exceeding boiling temperature for the solution, i.e approx. 100°C. A suitable interval is room temperature (20°C) up to 75 °C, suitably 30 to 60 °C, ideally 30 to 50 °C.

The treatment period could vary between 1 min and up to 24 hours, preferably 30 minutes up to 6 hours, most preferred 2 hours to 4 hours.

The release rates of metal from the alloy during the passivation treatment was followed by measuring the concentration of the metals in question in the bath liquid using ICP-AES (Inductive Coupled Plasma - Atomic Emission Spectroscopy). Data from the experiment are shown in Table 1

**Table 1**

| Metal | Metal release (µg/cm²/h) | SD |
|---|---|---|
| Co | 0,13 | 0,008 |
| Cr | 0,03 | 0,005 |
| Mo | 0,02 | 0,000 |
| Ni | 0,12 | 0,036 |

As can be seen, the release rate is considerably higher for Co and Ni (the largest alloy constituents) than for Cr and Mo. Of the total amount of metal released, Co accounts for 43%, Ni 40%, Cr 10% and Mo 7%. Thus, these results show preferential dissolution of Co and Ni during the passivation in the strong acidic solution. Relatively lower release rates of Cr and Mo may be a result of formation of stable oxides of these elements. When stainless steel is passivated in an acidic solution, the passive surface film becomes enriched in Cr, as a consequence of selective dissolution of Fe. Similarly, preferential dissolution of Co and Ni leads to a Cr enriched passive oxide film on the Co-base alloy.

### Example 2 (release of metal in synthetic biological media)

The release of Co, Ni, Cr and Mo from non-passivated and passivated 35N LT, respectively, was investigated by immersing lead coils in PBS (phosphate buffer saline) with 100 mM H₂O₂, a synthetic biological media. Total immersion time was 3 hours (180 minutes). The addition of H₂O₂ is done to take into account accelerated corrosion due to generation of aggressive species in the biological system during inflammatory response. The metal release rates are shown in Table 2, which compares passivated and non-passivated lead coils made of alloy 35N LT.

**Table 2**

| Metal | Metal release (µg/cm²/h) | SD |
|---|---|---|
| Co, pass. | 0,12 | 0,02 |
| Co, non-pass. | 0,23 | 0,04 |
| Cr, pass. | 0,17 | 0,01 |
| Cr, non-pass. | 0,19 | 0,06 |
| Mo, pass. | 0,09 | 0,00 |
| Mo, non-pass. | 0,13 | 0,01 |
| Ni, pass | 0,55 | 0,19 |
| Ni, non-pass. | 0,76 | 0,07 |

The table clearly shows that the passivation treatment resulted in a decrease in metal release, in particular of Co, from the alloy 35N LT in PBS + 100mM H₂O₂. This can be explained by the enrichment of Cr and depletion of Co in the passive oxide film provided by the passivation treatment.

Since MIO is believed to be caused by metal (notably Co) ions originating from the alloy, the results show that chemical passivation treatment according to the present invention is beneficial in reducing MIO in applications were metals are exposed to corrosive environments.

### Example 3

A lead coil as in Example 1 is submersed in a chromic acid bath for 30 minutes at 46 °C. A passivated surface is obtained.

### Example 4

A lead coil as in Example 1 is submersed in a chromic acid bath for 60 minutes at 56 °C. A passivated surface is obtained.

### Example 5

A lead coil as in Example 1 is submersed in a tricresyl phosphate (TCP) bath for 2 days at 107 °C. A passivated surface is obtained.

### Example 6

A lead coil as in Example 1 is exposed to citric acid solution typically 4-10% by weight. A passivated surface is obtained.

The resultant passivated, insulated lead can be connected to a cardiac pacemaker at a proximal end, inserted into a patient and connected to the patient's heart at a distal end.

The lead discussed herein offers improved resistance to degradation of the polymer insulation without possessing any statistically significant increase in product dimension. Furthermore, the flexibility, fatigue-resistance, glidability and other beneficial properties of the insulated lead are maintained. The passivated layer on the conductor thus provides the additional benefit of extending potential product life. Extending product life in a product such as a pacemaker lead reduces the risk of complications or injury to the patient while also reducing the chance that an additional procedure is required to remove and replace a lead, which also reduces the risk of adverse outcome for the patient while minimizing medical treatment costs.

## Claims

1. An implantable cardiac pacemaker lead, comprising:
an electrically conductive metal or metal alloy, comprising Co, forming an elongated conductor having an outer surface;
a passivated area on at least a portion of the metal or metal alloy outer surface, wherein the passivated area of the metal or metal alloy is depleted of Co; and
an insulating layer having an inner surface configured to fit over at least part of said passivated area.

2. An implantable cardiac pacemaker lead according to claim 1, wherein the passivated area is a film having a thickness of 1 nm to 20 nm.

3. An implantable cardiac pacemaker lead according to claim 1 or 2, wherein the passivated area of the metal or metal alloy is enriched in Cr.

4. An implantable cardiac pacemaker lead according to claim 1, wherein the insulating layer is polymer-based.

5. An implantable cardiac pacemaker lead according to claim 4, wherein the polymer is polyurethane.

## Patentansprüche

1. Implantierbare Herzschrittmacherleitung, die das Folgende umfasst:
ein elektrisch leitfähiges Metall oder eine elektrisch leitfähige Metalllegierung, umfassend Co, bildend einen länglichen Leiter, welcher eine äußere Oberfläche aufweist;
einen passivierten Bereich auf wenigstens einem Teil der äußeren Oberfläche des Metalls oder der Metalllegierung, wobei der passivierte Bereich des Metalls oder der Metalllegierung an Co verarmt ist; und
eine Isolationsschicht, die eine innere Oberfläche, welche so konfiguriert ist, dass sie über wenigstens einen Teil des passivierten Bereichs passt, aufweist.

2. Implantierbare Herzschrittmacherleitung nach Anspruch 1, wobei es sich bei dem passivierten Bereich um einen Film mit einer Dicke von 1 nm bis 20 nm handelt.

3. Implantierbare Herzschrittmacherleitung nach Anspruch 1 oder 2, wobei der passivierte Bereich des Metalls oder der Metalllegierung mit Cr angereichert ist.

4. Implantierbare Herzschrittmacherleitung nach Anspruch 1, wobei die Isolationsschicht polymerbasiert ist.

5. Implantierbare Herzschrittmacherleitung nach Anspruch 4, wobei es sich bei dem Polymer um Polyurethan handelt.

## Revendications

1. Conducteur implantable pour stimulateur cardiaque, comprenant :
un métal ou alliage de métal électriquement conducteur, comprenant du Co, formant un conducteur allongé ayant une surface extérieure ;
une zone passivée sur au moins une partie de la surface extérieure du métal ou de l'alliage de métal, la zone passivée du métal ou de l'alliage de métal étant appauvrie en Co ; et
une couche isolante ayant une surface intérieure configurée pour s'adapter sur au moins une partie de ladite zone passivée.

2. Conducteur implantable pour stimulateur cardiaque selon la revendication 1, dans laquelle la zone passivée est un film ayant une épaisseur de 1 nm à 20 nm.

3. Conducteur implantable pour stimulateur cardiaque selon la revendication 1 ou 2, dans laquelle la zone passivée du métal ou de l'alliage de métal est enrichie en Cr.

4. Conducteur implantable pour stimulateur cardiaque selon la revendication 1, dans laquelle la couche isolante est à base de polymère.

5. Conducteur implantable pour stimulateur cardiaque selon la revendication 4, dans laquelle le polymère est un polyuréthane.
